(19) Europäisches Patentamt / European Patent Office / Office européen des brevets

(11) **EP 1 988 984 B1**

(12) **EUROPÄISCHE PATENTSCHRIFT**

(45) Veröffentlichungstag und Bekanntmachung des Hinweises auf die Patenterteilung:
**07.09.2011  Patentblatt 2011/36**

(21) Anmeldenummer: **07703397.5**

(22) Anmeldetag: **12.02.2007**

(51) Int Cl.:
*B01F 3/04* (2006.01)     *B01F 11/00* (2006.01)
*C12M 1/06* (2006.01)     *C12M 1/12* (2006.01)
*C12M 3/02* (2006.01)     *C12M 3/06* (2006.01)

(86) Internationale Anmeldenummer:
**PCT/EP2007/001162**

(87) Internationale Veröffentlichungsnummer:
**WO 2007/098850 (07.09.2007 Gazette 2007/36)**

(54) **VERFAHREN UND VORRICHTUNG ZUR BE- UND ENTGASUNG VON FLÜSSIGKEITEN, INSBESONDERE IN DER BIOTECHNOLOGIE UND SPEZIELL VON ZELLKULTUREN**

PROCESS AND APPARATUS FOR INTRODUCTION OF GAS INTO AND DEGASSING OF LIQUIDS, IN PARTICULAR IN BIOTECHNOLOGY AND ESPECIALLY OF CELL CULTURES

PROCEDE ET DISPOSITIF POUR LE GAZAGE ET LE DEGAZAGE DE LIQUIDES, NOTAMMENT EN BIOTECHNOLOGIE ET PARTICULIEREMENT POUR DES CULTURES CELLULAIRES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **24.02.2006  DE 102006008687**

(43) Veröffentlichungstag der Anmeldung:
**12.11.2008  Patentblatt 2008/46**

(73) Patentinhaber: **Bayer Technology Services GmbH 51368 Leverkusen (DE)**

(72) Erfinder:
• **BROD, Helmut**
  **51061 Köln (DE)**
• **KAULING, Jörg**
  **51069 Köln (DE)**
• **FRAHM, Björn**
  **40699 Erkrath (DE)**
• **ROSE, Reinhold**
  **51371 Leverkusen (DE)**

(74) Vertreter: **Lütjens, Henning Bayer Technology Services GmbH BTS-LP/PL 51368 Leverkusen (DE)**

(56) Entgegenhaltungen:
EP-A1- 0 172 478     DE-A1- 3 535 183
DE-A1- 3 614 712     DE-A1- 3 809 163
DE-A1- 4 412 484     DE-A1-102004 029 709
DE-U1- 8 423 210     US-A- 4 289 854
US-A- 5 846 817     US-A1- 2002 139 748

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren und eine Vorrichtung zur blasenfreien Begasung von Flüssigkeiten, insbesondere in der Biotechnologie und speziell von Zellkulturen.

Die Begasung von Flüssigkeiten dient dem Eintrag und der Desorption von Gasen. Insbesondere bei der Versorgung und Vermehrung von Zellkulturen in Kulturmedien stellen die ausreichende Sauerstoffversorgung und Kohlendioxid-Entsorgung ein Problem dar.

Zellkulturen nehmen jedoch in der pharmazeutischen Industrie eine immer bedeutendere Position ein, z.B. bei der Herstellung von Antikörpern und Proteinen. Besonders zur Herstellung komplexerer Substanzen sind Zellkulturen prädestiniert, da sie die Fähigkeit aufweisen, hoch glykosylierte Proteine mit posttranslatorischen Modifikationen herzustellen.

Zellkulturen stellen besondere Anforderungen an die Reaktoren (und Behältnisse), in denen sie kultiviert werden. Zu vermeiden sind z.B. hohe Scherkräfte, da diese die Zellmembran der zellwandlosen Zellen schädigen. Scherkräfte entstehen beispielsweise an Rührorganen oder beim Zerplatzen von Gasblasen an der Flüssigkeitsoberfläche. Zu vermeiden ist ferner eine Schaumbildung, da Zellen dazu neigen, mit dem Schaum zu flotieren. In der Schaumschicht finden sie nicht adäquate Kultivierungsbedingungen vor. Der Einsatz von Antischaummitteln kann auch zu Zellschädigung oder Ausbeuteverlusten in der Aufarbeitung oder zu einem vermehrten Aufarbeitungsaufwand führen.

Die Oberflächenbegasung ist eine Art der Begasung von Zellkulturen, die einigen der oben skizzierten Anforderungen Rechnung trägt. Bei der Oberflächenbegasung erfolgt die Sorption- und Desorption von Gasen über die Oberfläche der Flüssigkeit, also der Grenzschicht zwischen Reaktor-Gasraum und Flüssigkeit. Da mit der Oberflächenbegasung abhängig vom Oberflächenzu Volumenverhältnis in der Regel nur ein geringes Flüssigkeitsvolumen begast werden kann, wird häufig die submerse Begasung verwendet. Diese kann in blasen- und blasenfreie Begasung unterschieden werden. Jegliche Arten der Begasung, bei denen Blasen auftreten, weisen jedoch die bereits aufgezeigten Nachteile wie Schaumbildung oder Zerplatzen der Gasblasen an der Flüssigkeitsoberfläche auf. Ferner ist eine Verteilung und/oder Dispergierung der Gasblasen notwendig, z.B. durch ein Rührorgan. Dieses erzeugt jedoch wiederum Scherkräfte. In der Regel lassen sich zwar die geforderten Gastransportraten mit Begasungen, welche Blasen erzeugen, erreichen. Diese führen aber gleichzeitig zu Zellschädigung.

Die blasenfreie Begasung löst die Problematik, indem der Gasaustausch über eine eingetauchte Membranfläche erfolgt. Hierbei wird die Begasung mit geschlossenen oder offenporigen Membranen durchgeführt. Diese sind z.B. in der durch einen Rührer bewegten Flüssigkeit angeordnet. Als Schlauchmaterial hat sich Silikon gegenüber porösen Polymeren durchgesetzt. Gründe hierfür sind die hohe Gaspermeabilität, die hohe thermische Beständigkeit und die homogen über die Länge der Schlauchsegmente von bis zu 50 m verteilten Schlaucheigenschaften, die auch nach einer Sterilisation erhalten bleiben. Die großen Schläuchlängen der Schlauchsegmente dienen der Verkürzung der zeitintensiven Herstellung der Schlauchstatoren. Im Allgemeinen wird der Silikonschlauch nach einmaliger Verwendung verworfen.

Nachteil der bisherigen Membranbegasungen ist der vergleichsweise geringe Stofftransportkoeffizient (H.-J. Henzler, J. Kauling: "Oxygenation of cell cultures" Bioprocess Engineering 9 (1993) S. 61-75). Um hohe Stofftransportraten zu erreichen ist es erforderlich, entsprechend viel Membranfläche im Bioreaktor zu installieren. Dies ist jedoch bezüglich Konstruktion und Handhabung aufwendig (Montage, Sterilisation, Reinigung, Erzeugung von unzureichend durchmischten Bereichen etc.). Des Weiteren kann der Leistungseintrag erhöht werden. Da der Stofftransportkoeffizient vom Leistungseintrag abhängig ist, kann hierdurch eine Steigerung der Stofftransportrate erzielt werden. Das Potential ist jedoch durch die resultierende Scherbelastung der Zellen aufgrund des höheren Leistungseintrages begrenzt.

Aus diesen Rahmenbedingungen resultiert die Anforderung, mit einer entsprechenden Membranbegasung hohe Stofftransportkoeffzienten bei gleichzeitig geringem Leistungseintrag bzw. gleichzeitig geringer Scherbelastung zu erreichen. Eine Nebenbedingung ist, dass die Durchmischung des Reaktorraumes weiterhin in ausreichendem Maße erfolgt. Diese muss einerseits die Sedimentation der Zellen verhindern, andererseits die Einmischung von Flüssigkeiten in ausreichend kurzen Mischzeiten ermöglichen.

Um dies zu erreichen wurden z.B. in EP 0172478 A1 ein Verfahren und eine Vorrichtung beschrieben, welche sich in der Praxis bislang nicht durchsetzen konnten. Hierbei handelt es sich um auf einen Korb aufgewickelte Membranschläuche, wobei der Korb im Inneren des Bioreaktors eine Exzenterbewegung ohne Eigenrotation (!) ausführt. Hierfür besteht aber die Notwendigkeit zu einer Exzentervorrichtung. Die mechanischen Vorrichtungen des Exzenters müssen zwangsläufig im Reaktor, also innerhalb des Sterilbereiches, vorgesehen werden. Dies führt einerseits zu Problemen bei der Sterilisation der Exzentervorrichtung, andererseits ist diese eine bleibende Gefahrenquelle für Kontaminationen des Sterilbereiches. Ferner ist es nicht sinnvoll, eine maschinentechnisch nicht simple Vorrichtung wie eine Exzentervorrichtung, die der Wartung bedarf, im Sterilbereich anzubringen. Es ist davon auszugehen, dass die Notwendigkeit der Exzentervorrichtung und die damit verbundenen Probleme die Anwendung dieses Patentes verhindert haben.

DE8423210U1 und DE3535183A1 offenbaren eine Vorrichtung zur blasenfreien Begasung von Flüssigkeiten, insbesondere von Kultumedien zur Vermehrung von Gewebekulturen. Die Begasung erfolgt mittels eines Membrankorbs aus röhrenförmigen Membranhohlfäden, der flexibel in dem zu begasenden Medium aufhängbar und pendelnd bewegbar ist. Eine Pendelbewegung des Membrankorbs führt zu einer eingeschränkten Begasung, da eine zu starke Pendelbewegung ein Herausschwappen der Flüssigkeit aus dem Begasungsbehälter verursachen könnte. Zudem scheint ein Rührer erforderlich, um eine ausreichende Anströmung der Membranflächen zu erzielen. Rührer sind aus den oben genannten Gründen bei scherempfindlichen Kulturmedien jedoch zu vermeiden.

DE3614712A1 offenbart eine Vorrichtung zur Kultivierung von Zellkulturen, bei welcher die Kulturen sich innerhalb eines flüssigen Nährmediums in einem geschlossenen Behälter befinden, wobei das Nährmedium dem Behälter zuführbar und aus diesem über ein zwischengeschaltetes Filter entfernbar ist.

In der Zellkulturflüssigkeit sind Microcarriers enthalten, auf denen die Zellen wachsen und die durch das Filter zurückgehalten werden sollen.

Das Filter weist wenigstens eine Durchtrittsfläche auf, in welcher ein flexibles Filtergewebe abdichtend, aber zugleich locker beweglich aufgehängt ist, wobei das Filter innerhalb des Nährmediums bewegbar ist. Durch die Bewegung des Filters soll erreicht werden, dass auch das innerhalb der Flüssigkeit im Behälter locker aufgehängte Filtergewebe in Bewegung gerät, wobei diese Bewegung zu einem Abfallen der gegen das Filter geführten Microcarriers führen und/oder das Anhaften der Microcarriers verhindern soll. Die Bewegung des Filters kann eine hin- und hergehende Bewegung oder auch eine Taumelbewegung sein.

DE3614712A1 lehrt, dass das Filter mit einem beweglichen Membrankorb verbunden werden kann, der Membranen für eine blasenfreie Begasung der Zellkulturen haltert und ebenfalls einer Bewegung innerhalb der Flüssigkeit unterworfen ist. Die Bewegung des Membrankorbs ist nicht näher spezifiziert.

Aufgabe der vorliegenden Erfindung war es folglich, ein Verfahren und eine Vorrichtung zur effektiven, schonenden, leicht zu reinigenden blasenfreien Begasung von Zellkulturen bereitzustellen.

Überraschend wurde nun gefunden, dass ein Verfahren gemäß Anspruch 1 sowie eine Vorrichtung gemäß Anspruch 7 zur Begasung von Flüssigkeiten, insbesondere von in der Biotechnologie verwendeten Flüssigkeiten und speziell von Zellkulturen, mit einem Gasaustausch über eine oder mehrere beliebig geartete, eingetauchte Membranflächen (Schläuche, Zylinder, Module etc.), die gestellte Aufgabe Lösen.

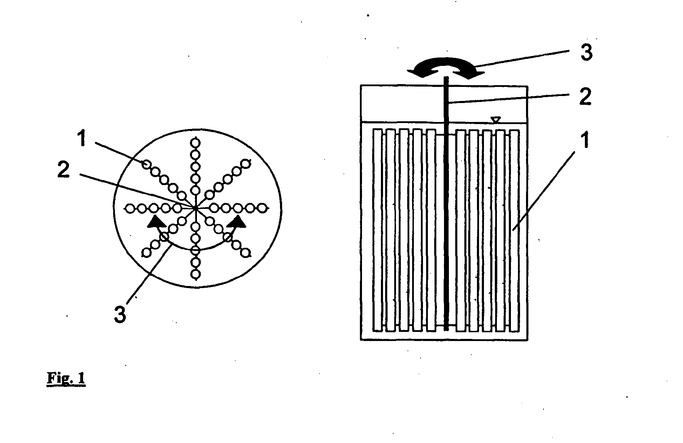

In Fig. 1 wird schematisch ein Beispiel für eine derartige Anordnung und Bewegung dargestellt, wobei die Membranfläche in diesem Fall durch Membranschläuche (1) gebildet wird, die an einer Rotorwelle (2) vertikal quer zur Rotationsrichtung (3) angeordnet sind.

Bei einer rotatorisch oszillierenden Bewegung bewegt sich die Membranfläche zunächst in die eine Rotationsrichtung, wobei die Bewegung beliebig gestaltet werden kann. Ein Beispiel ist die Beschleunigung der Membranfläche mit einer bestimmten Winkelbeschleunigung bis zu einer bestimmten Winkelgeschwindigkeit, mit der sich die Membranfläche dann für eine bestimmte Zeit bewegt. Anschließend wird die Membranfläche mit einer festgelegten Verzögerung bis zum Stillstand abgebremst. Es folgt, ggf. nach einer festgelegten Stillstandszeit, anschließend die Bewegung in die andere Rotationsrichtung. Diese Bewegung kann spiegelbildlich der vorher beschriebenen erfolgen oder andersartig gestaltet sein.

Die rotatorisch oszillierende Bewegung bietet dabei folgende Vorteile:

- Einfach zu realisieren über eine entsprechende Ansteuerung des Antriebsmotors.

- Vom konstruktionstechnischen und mechanischen Aspekt her gibt es keine Zusatzanforderungen im Gegensatz zu komplexeren Bewegungen wie z.B. denen in EP 0172478 A 1 offenbarten.

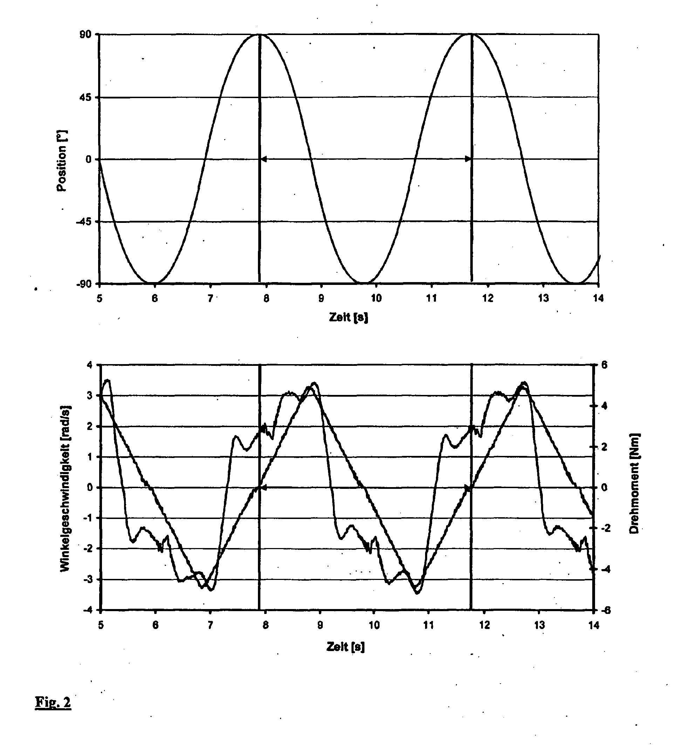

- Durch eine gezielte Abänderung der Bewegung kann die Bewegung so optimiert werden, dass die Anströmung der Membranfläche optimal ist. Da der Stofftransportkoeffizient von der Anströmung der Membranfläche abhängt, ist eine Bewegung optimal, bei der die Membranfläche jeweils eine möglichst hohe Relativgeschwindigkeit zur Flüssigkeit aufweist. Die Flüssigkeit wird jedoch auch durch die Membranfläche beschleunigt. Würde die Membranfläche nur in einer Richtung rotieren, so ergäbe sich nach einer gewissen Zeit eine Mitrotation der Flüssigkeit und demnach kein / nur noch wenig Stofftransport. Stromstörer wären erforderlich, um eine Relativgeschwindigkeit zwischen Membranfläche und Flüssigkeit zu erzeugen. Bei der rotatorisch oszillierenden Bewegung hingegen wird eine dauerhafte Mitrotation der Flüssigkeit durch die periodische Bewegungsumkehr der Membranfläche verhindert. Eine sinnvolle Bewegung sieht beispielsweise wie folgt aus: Beschleunigung der Membranfläche derart, dass die Relativgeschwindigkeit zur Flüssigkeit möglichst hoch bei gleichzeitig geringem Leistungseintrag / geringer Scherbeanspruchung ist. Sofern eine weitere Beschleunigung der Membranfläche nicht sinnvoll ist, z.B. weil diese zu hohe Geschwindigkeiten erreicht oder die Flüssigkeit in unerwünschtem Maße mitrotiert, wird die Membranfläche verzögert. Hierbei ist die Bewegung so vorzugeben, dass wiederum die Relativgeschwindigkeit zur Flüssigkeit möglichst hoch bei gleichzeitig geringem Leistungseintrag / geringer Scherbeanspruchung ist. Bis auf einen möglichst kurzen Zeitraum, in der die Membranfläche beim Verzögern die Winkelgeschwindigkeit der Flüssigkeit innehat, ist ansonsten immer eine hohe Relativgeschwindigkeit präsent. Bei der anschließenden Bewegungsumkehr und Abfahren der spiegelbildlichen Bewegung entsteht wiederum der entsprechende Sachverhalt. Dabei ist auch beim Stillstand der Membranfläche bei der Bewegungsumkehr noch eine Relativgeschwindigkeit vorhanden, da die Flüssigkeit langsamer als die Membranfläche verzögert wird und noch "nachläuft". Ein derartiger Bewegungsablauf ist in Fig. 2 bezüglich Position von sternförmig angebrachten Membranschläuchen, deren Winkelgeschwindigkeit und dem Drehmoment zur Erzeugung dieses Bewegungsablaufes dargestellt. Das obere Diagramm in Fig. 2 zeigt als Beispiel die Position des Rotors, auf den die Membranschläuche gewickelt sind. Der markierte Zeitraum (ca. Sekunde 8-12) entspricht einem rotatorisch oszillierenden Bewegungsablauf. Hier vollführt der Rotor eine Bewegung um 180° in die eine Rotationsrichtung und wieder zurück. Die zugehörige Winkelgeschwindigkeit ist in dem unteren Diagramm von Fig. 2 dargestellt. Dies zeigt ferner das an der Antriebsachse gemessene Drehmoment, wobei das Leerdrehmoment subtrahiert worden ist. Als Leerdrehmoment wird das Drehmoment bezeichnet, welches zum Antrieb des Rotors mit dem gleichen Bewegungsablauf in dem gleichen Behältnis, aber ohne Flüssigkeit, erforderlich ist. Das aufgetragene Drehmoment entspricht also dem Drehmoment, welches auf die Flüssigkeit einwirkt. Es kann mit der Umströmung der Membranfläche korreliert werden. Der Verlauf des Drehmomentes hat in diesem Beispiel annähernd Kastenform. So weist das Drehmoment für einen gewissen Zeitraum annähernd gleiche Werte auf, bis es dann innerhalb von kürzester Zeit das Vorzeichen wechselt. Dies veranschaulicht, dass die Umströmung der Membranfläche bis auf den kurzen Zeitraum, in dem das Vorzeichen des Drehmomentes wechselt, gleichmäßig ausgebildet und gut ist.

- Ein weiterer Vorteil der rotatorisch oszillierenden Bewegung der Membranfläche ist die Tatsache, dass ein separates Rühr- oder Mischorgan zur Erzeugung einer Anströmung der Membranfläche entfällt. Diese Vereinigung von Membranflächenbereitstellung und Erzeugung der Membranflächenanströmung vermeidet Zonen von lokal hohem Leistungseintrag und lokal hoher Scherbeanspruchung. Diese entstehen sonst z.B. hinter den äußeren Enden der Rührerblätter. Ferner erfolgt die Anströmung der Membranfläche bei anderen Systemen zeitlich und örtlich unregelmäßig, indem die Rührerblätter periodisch an den Membranflächen an der einen Seite vorbeistreichen. Bei der hier beschriebenen Erfindung dagegen erfolgt der Leistungseintrag räumlich gleichmäßiger und dient unmittelbar der Anströmung der Membranfläche.

- Durch die erfindungsgemäße Ausgestaltung des Verfahrens und der Vorrichtung wird eine gezielte Flüssigkeitsbewegung an allen Stellen der Membranfläche und damit ein hoher, definierter Stofftransport infolge der Bewegung der Membranflächen relativ zur Flüssigkeit erzeugt.

- Insgesamt wird durch das erfindungsgemäße Verfahren und die erfindungsgemäße Vorrichtung also ein besseres Verhältnis von Stofftransport und dem zur Erzeugung des Stofftransports erforderlichen mechanischen Leistungseintrag erzielt als bei herkömmlichen Verfahren und Vorrichtungen.

Eine bedarfsabhängige Steuerung der Sauerstoffversorgung kann durch die Änderung der rotatorisch oszillierenden Bewegung erreicht werden. Die Variation der Bewegung bewirkt eine veränderte Umströmung der Membranflächen, was wiederum eine Veränderung des Stofftransportes bewirkt.
Die bedarfsabhängige Steuerung der Sauerstoffversorgung kann ferner durch eine Änderung der Gaskonzentration und/oder des Drucks des in den Raum innerhalb der Membranfläche einströmenden Gases oder Gasgemisches oder einer Gaskomponente gesteuert werden. Analog ergibt sich die Steuerungsmöglichkeit beim Ausströmen aus dem Raum innerhalb der Membranfläche.
Bei Beaufschlagung mit entsprechendem Druck kann die Membranfläche auch zur Erzeugung von Mikroblasen oder Gasblasen in der Flüssigkeit genutzt werden. Dies ist bei robusten Zelllinien, welche eine Blasenbegasung tolerieren, von Vorteil. Hierdurch kann der Stoftransportkoeffizient erhöht werden.
Als Membranflächen eignen sich z.B. nicht poröse Silikonschläuche in verschiedenen Durchmessern und Wandstärken. Vorzugsweise liegen diese in einem Bereich von Innendurchmesser ~1 mm bei einem Außendurchmesser von ~1,4 mm bis zu einem Innendurchmesser von ~2 mm bei einem Außendurchmesser von ~3 mm. Die Parameter Schlauchdurchmesser und Schlauch gesamtlänge sollten so gewählt werden, dass ein ausreichender Stofftransport für die Applikation gewährleistet ist.
Der Stofftransport wird u. a. von dem Verhältnis von Membranoberfläche zu Reaktorflüssigvolumen bestimmt (volumenspezifische Stoffaustauschfläche). Hier sind Werte von 25 m$^{-1}$ bis 45 m$^{-1}$ für tierische Zellkulturen gebräuchlich. In der vorliegenden Erfindung erreicht die volumenspezifische Stoffaustauschfläche Werte zwischen 0,1 m$^{-1}$ und 150 m$^{-1}$, bevorzugt werden 1 m$^{-1}$ bis 100 m$^{-1}$ und besonders bevorzugt 5 m$^{-1}$ bis 75 m$^{-1}$ erreicht.
Der Stofftransport wird jedoch ferner bestimmt von dem Stofftransportkoeffizienten von der Gasphase im Schlauch zur Flüssigphase um den Schlauch herum und dem entsprechenden treibenden Konzentrationsgefälle. Der sich daraus ergebende Betriebsparameter Membraninnendruck ergibt sich aus den gewünschten Stoffströmen durch die Membran und wird nach oben hin durch zwei Grenzen beschränkt. Einerseits lässt die Materialbelastbarkeit des Silikonschlauches nur einen bestimmten Membraninnendruck zu, andererseits tritt womöglich bereits bei geringerem Druck Blasenbildung in nicht gewünschtem Umfang auf der Membranaußenoberfläche auf. Der Membraninnendruck entsteht durch das entsprechende Einstellen der folgenden Parameter: Volumenstrom durch die Membran bzw. Druck am Anfang des Membranschlauches und Druck am Ende des Membranschlauches. Der andere sich ergebende Betriebsparameter Gaskonzentration in der Flüssigphase ergibt sich aufgrund der Betriebsbedingungen bzw. der Kultivierungsbedingungen.

Die in der Zellkulturtechnik übliche Einstellung des pH-Wertes über ein Puffer-$CO_2$-Gleichgewicht lässt sich üblicherweise durch Zumischen der notwendigen $CO_2$-Menge in das Eintrittsgas erreichen.

Das erfindungsgemäße Verfahren und die erfindungsgemäße Vorrichtung eignen sich auch in vorteilhafter Weise für ein blasenfreies Begasen von Microcarrier-Kulturen.

Das erfindungsgemäße Verfahren und die erfindungsgemäße Vorrichtung eignen sich auch in vorteilhafter Weise für die Anwendung von Dialyse, z.B. zur optimierten Prozessführung von Fermentationen. Die bei Fermentationen entstehenden Stoffwechselprodukte und/oder Nebenprodukte können per Dialyse abgeführt werden. Manche Stoffwechselprodukte und/oder Nebenprodukte sind teilweise unerwünscht, da sie in bestimmten Konzentrationsbereichen toxisch wirken oder sich in bestimmten Konzentrationsbereichen nachteilig auf die Fermentation auswirken. Dies kann z.B. durch eine Hemmung des Wachstums oder der Produktbildung erfolgen. Ferner müssen Stoffwechselprodukte und/oder Nebenprodukte, gegebenenfalls in Downstream Processing vom eigentlichen Produkt abgetrennt werden, wodurch sich Aufwand und Kosten erhöhen. Die Dialyse bietet die Möglichkeit, Stoffwechselprodukte und/oder Nebenprodukte aus dem Kultivierungsraum abzuführen (R. Pörtner, H. Märkl: "Dialysis cultures" Applied Microbiology and Biotechnology 50 (1998) S. 403-414). Über die Dialysemembran können niedermolekulare Komponenten wie z.B. Metabolite diffundieren, während die Zellen und das Produkt im Kultivierungsraum zurückgehalten werden. Auf der dem Kultivierungsraum gegenüberliegenden Seite der Dialysemembran befindet sich Dialysat. Über einen (kontinuierlichen) Austausch des Dialysates werden die niedermolekularen Komponenten abgeführt. Des Weiteren bietet sich auch die Möglichkeit Komponenten (z.B. Substrat) im Dialysat zu höheren Konzentrationen als im Kultivierungsraum einzubringen. Dadurch können. Komponente gezielt in den Kultivierungsraum nachdosiert werden. Dialysemembranen zu obig beschriebenen Anwendungen von Dialyse können, z.B. in Form von Modulen, auf dem Rotor angebracht werden. Diese Anbringung kann z.B. statt einer oder mehrerer Membranflächen oder auch zusätzlich zu diesen erfolgen.

Die erfindungsgemäße Vorrichtung ist durch die im Folgenden aufgeführten Merkmale charakterisiert:

Die Vorrichtung besteht aus einem drehbar gelagerten Rotor, der im Inneren des Behältnisses, z.B. einem Bioreaktor, bewegt werden kann. Der Rotor ist so ausgestaltet, dass er im Inneren des Bioreaktors Membranflächen wie zum Beispiel Schläuche, Zylinder, Module etc. tragen kann.

Der drehbar gelagerte Rotor kann von außerhalb des Bioreaktors durch einen Antrieb in eine rotatorisch oszillierende Bewegung versetzt werden. Die Übertragung des erforderlichen Antriebsdrehmoments vom Antrieb auf den Rotor im Inneren des Reaktors kann entweder über eine Magnetkupplung erfolgen, oder die Rotorwelle wird über eine drehende Abdichtung durch das Gehäuse des Bioreaktors geführt und direkt an den Antrieb gekuppelt. Die Verwendung einer Magnetkupplung ist aus steriltechnischer Sicht besonders vorteilhaft, weil sie sterile und insterile Räume eindeutig und ohne drehende Dichtung voneinander trennt.

Als Antrieb zur Erzeugung einer rotatorisch oszillierenden Bewegung muss die vom Motor zur Verfügung gestellte Leistung ausreichen, um mit dem Rotor trotz des Massenträgheitsmomentes des Rotors und des Fluids eine oszillierende Bewegung mit vorgegebenem Bewegungsablauf durchzuführen. Für die Auslegung des Antriebes ist also sowohl das Massenträgheitsmoment des Rotors als auch die Krafteinwirkung vom Fluid auf den Rotor entscheidend. Bei ausreichender Drehzahl des Motors bietet ein Getriebe die Möglichkeit, das erforderliche Drehmoment bereitzustellen. Als Antriebskonfiguration kommt zum Beispiel ein Exzentertrieb in Frage. Ein Exzentertrieb verwandelt die gleichförmige Rotation eines konventionellen Antriebsmotors in eine rotatorisch oszillierende Bewegung an der Abtriebswelle um. Daneben kommen als Antriebskonfiguration für die erfindungsgemäße Vorrichtung auch frei programmierbare Positionierantriebe in Frage, wie zum Beispiel ein Schrittmotor. Der Vorteil solcher frei programmierbarer Antriebssysteme liegt darin, dass die rotatorisch oszillierende Bewegung der Membranen in weiten Bereichen den Erfordernissen des Prozesses angepasst werden kann, während ein Exzentertrieb in der Regel nur eingeschränkte Verstellmöglichkeiten aufweist.

Parameter des Antriebs wie Drehzahl, Drehmoment und Getriebeuntersetzungen sind für die jeweilige Anwendung frei wählbar und vom Maßstab abhängig. Für Anwendungen im Bereich Biotechnologie werden die Parameter üblicherweise so gestaltet, dass sich ein volumenspezifischer Leistungseintrag von 0,01 W pro $m^{-3}$ bis zu 4000 W pro $m^{-3}$ Flüssigvolumen, bevorzugt um 1000 W pro $m^{-3}$, ergibt.

Für Zellkulturen beträgt der volumenspezifische Leistungseintrag üblicherweise 0,01 bis zu 100 W pro $m^{-3}$.

Weiterhin sollte die Parametergestaltung so erfolgen, dass sich für die Zellkulturapplikation maximale Relativgeschwindigkeiten zwischen Rotor und Flüssigkeit von 1 m s$^{-1}$, besser 0,15 m s$^{-1}$ ergeben.

Um die Spannungen aus der Verbindung Getriebe und Rotor zu absorbieren, wird das Getriebe üblicherweise mit dem Rotor über eine beliebige torsionssteife Kupplung verbunden, welche geringen Wellenversatz oder geringes Nicht-Fluchten der Wellen aufnimmt, z.B. eine Faltenbalg-Kupplung.

Die Vorrichtung zur Anbringung der Membranfläche kann in vorteilhafter Weise in ihrer Ausbildung leicht den besonderen Verhältnissen in Zellkulturen, z.B. Zellagglomeration, angepasst werden. Dies kann beispielsweise durch die Art und Anordnung der Membranflächen erfolgen.

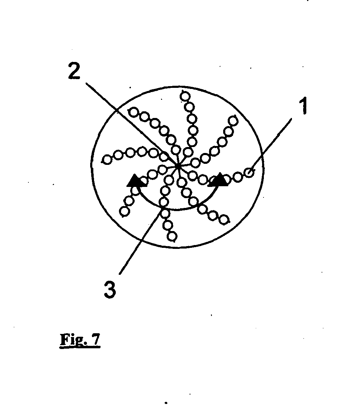

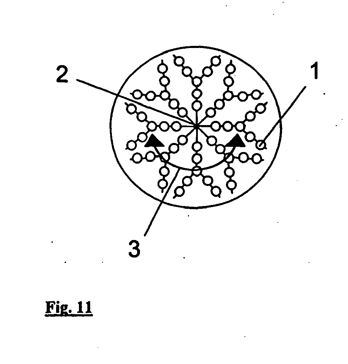

Der Rotor weist die für die Applikation erforderliche Anzahl von Rotorarmen auf, dies sind je nach Applikation 1 bis 64, bevorzugt 2 bis 32 und besonders bevorzugt 4 bis 16 Rotorarme. Die Problematik bei der Wahl der Rotorarmanzahl wird später genauer erläutert. Die Rotorarme können linear (z.B. Fig. 1, 8, 9, 10) oder verzweigt, in diesem Fall bevorzugt linear oder Y-förmig, (z.B. Fig. 11) sein und werden vorzugsweise sternförmig auf einem Halter angeordnet. Neben der sternförmigen Anordnung sind beliebige weitere Anordnungen denkbar. Vorteile von sternförmigen Anordnungen bzw. verzweigt sternförmigen Anordnungen oder gebogen sternförmigen Anordnungen (z.B. Fig. 7) sind eine recht gleichmäßige Verteilung der Membranfläche im Flüssigkeitsvolumen, eine gute Anströmung der Membranfläche und eine gute Durchmischung. Die Rotorarme werden symmetrisch oder asymmetrisch auf der Rotorwelle montiert und innerhalb des Reaktorraums angeordnet. Auf jedem Rotorarm wird die Membranfläche, bevorzugt die Membranschläuche, in regelmäßigen bzw. unregelmäßigen Abstand befestigt, z.B. durch Wickeln, Einhängen, Schnappverschlüsse oder durch andere literaturbekannte Methoden.

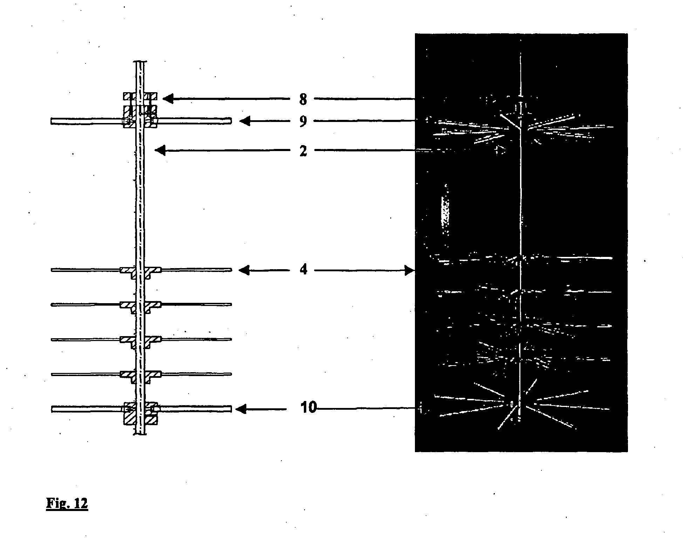

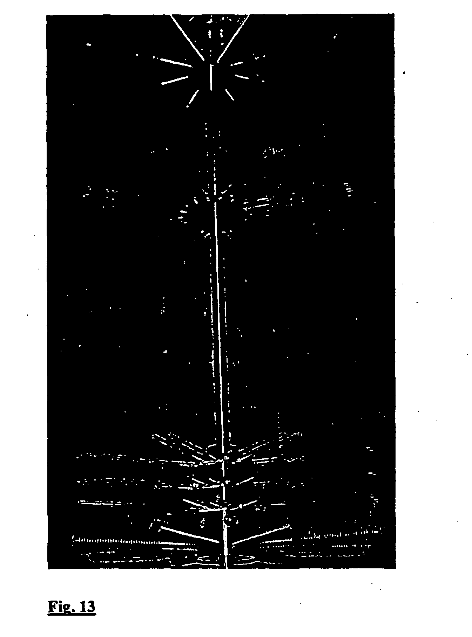

In einer besonderen Gestaltung der Vorrichtung bilden zwei Wickelarme einen Rotorarm. Auf diese Wickelarme wird die Membranfläche, bevorzugt die Membranschläuche, horizontal oder vertikal (z.B. Wickelarme an 9, 10, in Fig. 12 für vertikales Wickeln) in regelmäßigem (siehe Fig. 13) bzw. unregelmäßigem Abstand gewickelt (entsprechend Fig. 13, wobei nicht jede Vertiefung der Rotorarme mit einem Membranschlauch belegt ist).

Erfolgt jetzt eine Drehung des Rotors, so werden die Membranschläuche durch das Fluid im Reaktor bewegt und dadurch tangential angeströmt. Hinsichtlich der Anströmung der Membranschläuche ist zu beachten, dass die Anströmung bei gleicher Winkelgeschwindigkeit in Abhängigkeit von der Position des Membranschlauches mit zunehmender radialer Entfernung von der Rotorwelle im Allgemeinen besser wird. Grund hierfür ist die gleichermaßen zunehmende Umfangsgeschwindigkeit. Bevorzugt werden möglichst viele Membranschläuche möglichst weit außen bei guter Anströmung installiert. Eine Möglichkeit, diesem Anspruch gerecht zu werden, besteht darin, die Anzahl der Rotorarme um die Welle zu erhöhen. Negativ wirkt sich eine Erhöhung der Anzahl der Arme allerdings sowohl auf die Durchmischung als auch auf die Anströmung der Membran aus (Schaffung von weniger durchmischten Kompartimenten zwischen den Armen). Hinzu kommt, dass unter der zunehmenden Anzahl der Arme die Handhabung des Rotors beim Auf- und Abwickeln der Schläuche sowie beim Ein- und Ausbau leidet. Auch die Befestigung der Arme an der Welle gestaltet sich mit größerer Anzahl der Arme aus Platzgründen zunehmend problematisch.

Die Versorgung der rotatorisch oszillierenden Membranfläche für die Zu- und Abfuhr von Gas erfolgt vorzugsweise von der nicht bewegten Umgebung aus, z.B. dem Reaktordeckel, mit einer Drehdichtung oder mit Hilfe von flexiblen Schläuchen. Drehdichtungen sind in der Zellkulturtechnik meist unerwünscht, da sie Schwierigkeiten beim Reinigen und der Sterilisation bereiten können. Dies ist ein Vorteil der erfindungsgemäßen Vorrichtung gegenüber einer Vorrichtung, bei der die Membranflächen rein rotatorisch, d.h. ohne Umkehr der Bewegungsrichtung bewegt werden. Ohne Umkehr der Bewegungsrichtung würden sich die Schläuche mit zunehmender Umdrehung immer stärker tordieren und schließlich abreißen. Bei rotatorisch oszillierenden Membranflächen findet aufgrund der Hin- und Herbewegung keine Netto-Torsion der flexiblen Schläuche statt. Voraussetzung ist natürlich die Gestaltung der Hin- und Herbewegung derart, dass die Membranflächen sich nach Abschluss einer Periode der Bewegung am Ausgangspunkt der Bewegung befinden.

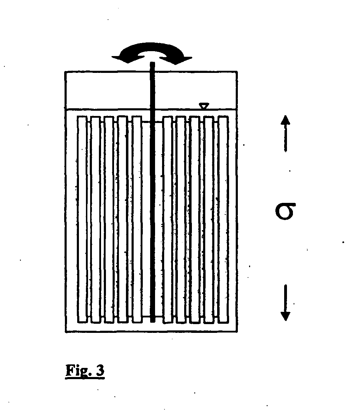

Ein weiterer Vorteil der Vorrichtung mit gewickelten Membranschläuchen ist, dass die Spannung σ der Membranfläche, z.B. der Membranschläuche variiert werden kann (Fig. 3). Die optimale Spannung ergibt sich anhand der Parameter Druck des in den Raum innerhalb der Membranfläche einströmenden Gases oder Gasgemisches, Druck des aus dem Raum innerhalb der Membranfläche ausströmenden Gases oder Gasgemisches und Geometrie, Strömungswiderstand und Deformation des Raumes innerhalb der Membranfläche (bei einem Membranschlauch z.B. Eingangsdruck, Ausgangsdruck, Innendurchmesser, Anzahl und Geometrie der Krümmungen des Membranschlauches sowie die Deformation der Krümmungen) (H. N. Qi, C. T. Goudar, J. D. Michaels, H.-J. Henzler, G. N. Jovanovic, K. B. Konstantinov: "Experimental and Theoretical Analysis of Tubular Membrane Aeration for Mammalian Cell Bioreactors" Biotechnology Progress 19 (2003) S. 1183-1189). Bei Membranschläuchen führt die Reduktion der Schlauchspannung zu einer verstärkten Auslenkung der Schläuche während der Bewegung. Eine größere Auslenkung der Schläuche verbessert deren Umströmung und damit den Stofftransportkoeffizienten. Die Spannung ist je nach Art der Applikation so zu wählen, dass die Membranschläuche einerseits langzeitstabil befestigt sind, sich andererseits aber vorzugsweise in der Strömung bewegen und um einige mm auslenken können. Dieser Vorteil kann mit einem herkömmlichen Membranbegasungssystem bestehend aus einem Stator, der die Membranflächen trägt, und einem Rotor, der für die Bewegung der Flüssigkeit sorgt, nicht oder nur kaum ausgenutzt werden, weil bei zu geringer Spannung der Membranschläuche die Gefahr besteht, dass diese mit dem Rotor in Kontakt kommen und zerstört werden. Bei der erfindungsgemäßen Vorrichtung kann ein solcher Schaden nicht auftreten, da es nur ein einziges bewegtes Teil im Reaktor gibt, welches gleichzeitig die Membranfläche selbst trägt.

In der besonderen Ausführungsform der Vorrichtung lässt sich die Schlauchspannung durch Vergrößerung des vertikalen Abstandes zwischen den Vorrichtungen zur Aufnahme der Wickelarme variieren (vgl. Fig. 12). Eine Feineinstellung der Membranschlauchspannung wird z.B. über die Verdrehung der Schrauben in der Spannvorrichtung (8) ermöglicht.

Aus der Reduzierung der Schlauchspannung ergibt sich das Problem der Fixierung der Membranschläuche auf den Wickelarmen. Eine große Krafteinwirkung auf die Membranschläuche könnte bei geringerer Schlauchspannung zum Abgleiten der Membranschläuche von den Wickelarmen führen. Um diesem Problem zu begegnen, wird z.B. die Oberfläche der Wickelarme mit einem Außengewinde versehen. Alternativ können z.B. außen an den Wickelarmen Stege vorgesehen werden, die ein Abrutschen der Schläuche außen von den Armen verhindern. Hierbei ist darauf zu achten, dass die aufgewickelten Membranschläuche durch etwaige Grate des Gewindes nicht zu Schaden kommen. Ferner bietet das Außengewinde auf den Wickelarmen des Sternhalters die Möglichkeit, die Schlauchwicklung zu variieren. Bei der Aufwicklung der Schläuche könnte z.B. nur jede zweite oder dritte Gewindevertiefung genutzt werden. Hierdurch ist die Einstellung eines definierten Abstandes zwischen den einzelnen Membranschläuchen möglich.

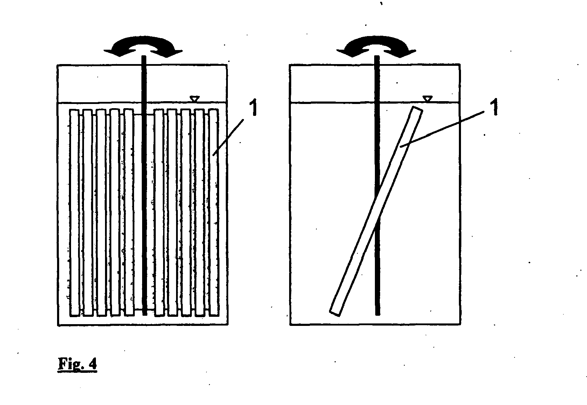

Schon bei dem oben erwähnten Patent EP 0172478 A1 zeigt sich, dass die Durchmischung bei einem derartigen System nicht unproblematisch ist. Hinsichtlich der Durchmischung ist die Bewegungsführung bei dieser Erfindung aufgrund der oszillierenden Rotorbewegung günstiger. Eine Möglichkeit, die axiale Durchmischung zu forcieren, ist das Anstellen der Membranfläche (Fig. 4).

Hierdurch wird insbesondere der reaktorinterne Austausch der Flüssigkeit in Richtung parallel zur Drehachse der bewegten Membranen verbessert. Durch beliebiges radiales Verdrehen von einer Vorrichtung (z.B. 9 oder 10 aus Fig. 12) lässt sich beispielsweise der Anstellwinkel der Membranschläuche verändern. Seitens der Konstruktion sollen vorzugsweise die Vorrichtungen (9, 10) unabhängig voneinander und variabel gegeneinander verdreht werden können.

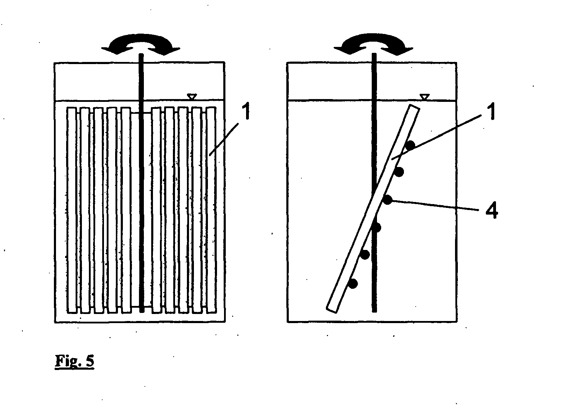

In einer weiteren Gestaltung der Vorrichtung werden Richtelemente auf der Rotorwelle montiert (vgl. (4) in Fig. 5 und 6 sowie 12). Diese Richtelemente sind entweder wie Wickelarme aufgebaut oder bestehen aus zwei Stangen. Sie werden innerhalb des Reaktorraums so angeordnet, dass sie die Schläuche entsprechend einseitig abstützen oder ausformen.

Eine weitere Möglichkeit, die Durchmischung zu verbessern, ist durch die Richtelemente dadurch gegeben, dass die durch den Strömungswiderstand bedingte Auslenkung der Membranfläche in einer Rotationsrichtung begrenzt wird (z.B. mittels Richtelemente (4) in Fig. 5). Dadurch ist die Auslenkung der Membranfläche in einer Rotationsrichtung stärker als in der entgegengesetzten Richtung, was in einer schwächeren Förderung der Flüssigkeit in dieser Richtung resultiert. Die ungleiche Förderung der Flüssigkeit in die beiden Bewegungsrichtungen des drehend oszillierenden Rotors führt zu einer Netto-Förderung in einer Richtung und damit zu einer besseren Durchmischung der Flüssigkeit.

Die Richtelemente, mit denen die Auslenkung der Membranflächen in einer Drehrichtung begrenzt wird, können gleichmäßig oder ungleichmäßig über die Länge der Membranschläuche verteilt sein. Diese Elemente werden so im Reaktor angeordnet, dass der gewünschte Effekt erzielt wird (siehe im Kapitel ‚Beispiel' den dargestellten Versuchsaufbau und die präsentierten Messergebnisse). Wenn sich diese Vorrichtungen zum Beispiel nur, im unteren Drittel des Reaktors befinden, wird der Strömungswiderstand auf unterschiedlichen Höhen unterschiedlich sein. Hierdurch ergibt sich bei der oszillierenden Drehbewegung der erfindungsgemäßen Vorrichtung eine zusätzliche Durchmischung der Flüssigkeit in Richtung parallel zur Drehachse.

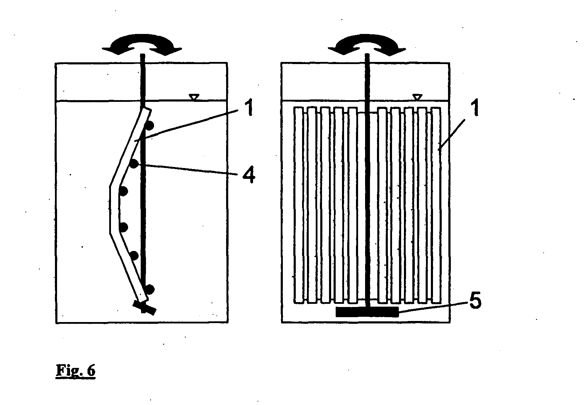

Neben der Möglichkeit, die Membranschläuche einseitig gegen Auslenkung abzustützen (vgl. auch Fig. 5), besteht eine weitere Möglichkeit, die Durchmischung zu fördern, in der Ausformung der Membranschläuche (z.B. einseitig bauchförmig, vgl. auch Fig. 6). Durch die Ausformung wird eine Asymmetrie der Strömungsbilder beider Bewegungsrichtungen geschaffen. Konstruktionstechnisch erlaubt beispielsweise der Einsatz der Richtelemente (4) aus Fig. 12 die Abstützung bzw. Ausformung. Die Richtelemente (4), mit denen die Auslenkung der Membranflächen in einer Drehrichtung begrenzt wird, können gleichmäßig oder ungleichmäßig über die Länge der Membranschläuche verteilt sein. Diese Vorrichtung wird vorzugsweise so gestaltet, dass die Richtelemente sowohl in der Höhe als auch in der Ausrichtung variabel montierbar sind. Z.B. durch Lösen bzw. Befestigen zweier Madenschrauben lassen sie sich beliebig an der Welle platzieren und fixieren.

In einer weiteren Ausgestaltung der Erfindung können der Stofftransport und die Durchmischung zusätzlich durch Anbringung von unbeweglichen Stromstörern im Inneren des Reaktors erhöht werden. Diese stören die Strömung, welche sich durch die rotatorisch oszillierende Bewegung der Membranflächen ausbildet.

In einer besonderen Ausgestaltung der Vorrichtung wird die Durchmischung dadurch verbessert, dass die oszillierend rotierende Membranvorrichtung keine Rotationssymmetrie aufweist. So können zum Beispiel bei der sternförmigen Anordnung von Rotorarmen in Fig. 1, auf welche die Membranflächen gewickelt sind, eine oder mehrere der Arme weglassen werden, so dass eine Lücke entsteht. Durch diese Brechung der Rotationssymmetrie wird naturgemäß die Durchmischung der Flüssigkeit im Reaktor verbessert. Ein weiterer Vorteil dieser unsymmetrischen Anordnung ist die Möglichkeit, in der entstehenden Lücke Sensoren, Tauchrohre etc. unterzubringen, ohne dass dadurch die Bewegung der Begasungsvorrichtung behindert wird. Dies gilt natürlich nur solange, wie die Amplitude der Oszillation der Membran gering genug ist, so dass es nicht zum Kontakt zwischen den eingetauchten Vorrichtungen (Sensoren, Tauchrohre etc.) und der Membran kommt.

Zur weiteren Verbesserung der Durchmischung, insbesondere um sicherzustellen, dass Partikel (Microcarrier, Zellen oder Zellagglomerate) sicher suspendiert werden, kann die Membranfläche der oszillatorisch rotierenden Vorrichtung zusätzlich zur Membranfläche auch mit Strömungsführung- und Durchmischungselementen wie z.B. Rührerblättern, Paddeln oder sonstigem ausgestattet werden, insbesondere in der Nähe des Reaktorbodens, um die Sedimentation dieser Partikeln zu verhindern (vgl. Rührer (5) in Fig. 6).

Eine weitere Möglichkeit, die Durchmischung zu verbessern, besteht darin, die Rotorarme um die Rotorwelle in einer der Rotationsrichtungen gebogen auszuführen (Fig. 7). Hierdurch wird bei Rotation in beiden Rotationsrichtungen die radiale Durchmischung forciert.

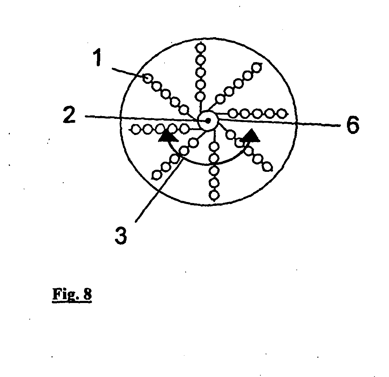

Die Durchmischung kann ferner dadurch verbessert werden, dass die Rotorarme tangential um die Rotorwelle in einer der Rotationsrichtungen an einer Vorrichtung (vgl. (6) in Fig. 8) angebracht werden. Auch hier wird bei Rotation in beiden Rotationsrichtungen die radiale Durchmischung forciert. Außerdem wird die Durchmischung im Bereich der Rotationsachse dadurch verbessert, dass aufgrund der tangentialen Anordnung der Rotorarme hier automatisch ein zylindrischer Bereich frei von Membranfläche entsteht.

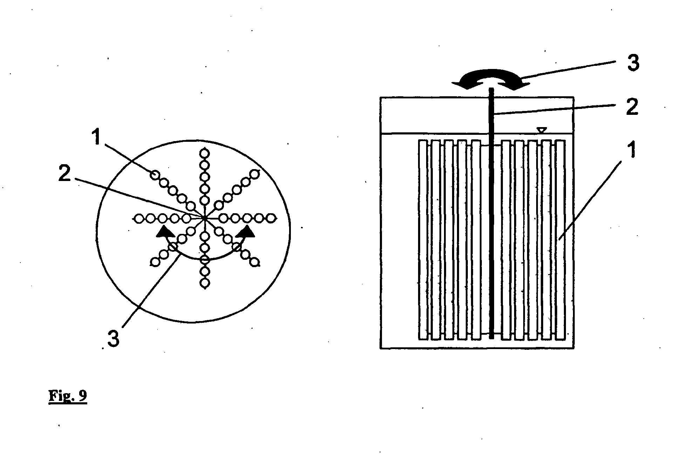

Eine andere Möglichkeit, die Durchmischung zu verbessern, besteht darin, die Rotorwelle exzentrisch im Behältnis anzubringen (Fig. 9). Gründe sind die Strömungsasymmetrie in Kombination mit einem Bereich frei von Membranfläche.

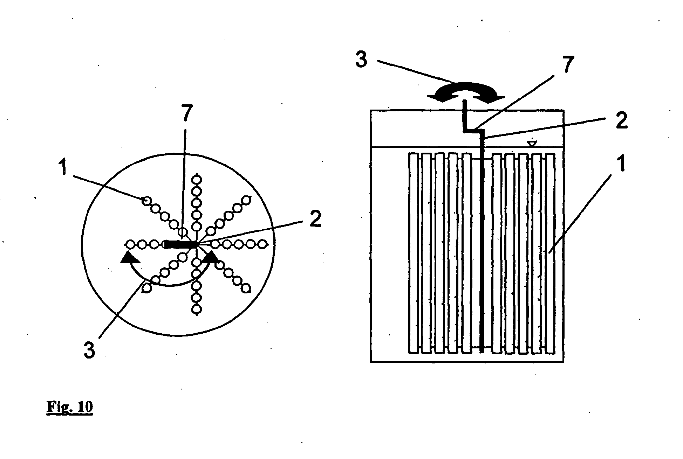

Unabhängig davon kann die Durchmischung verbessert werden, indem die Rotorwelle mit den beiden Rotationsrichtungen zwar zentrisch im Behältnis angebracht wird, dann aber einen Exzenter (vgl. (7) in Fig. 10) aufweist. Gründe hierfür sind die Strömungsasymmetrie in Kombination mit einem variablen Bereich frei von Membranfläche, der sich bei der Bewegung des Rotors permanent verändert.

Anders als bei herkömmlichen Vorrichtungen zur Membranbegasung von Flüssigkeiten besteht bei der erfindungsgemäßen Vorrichtung die Möglichkeit, die Membranfläche pro Volumen möglichst gleichmäßig im Reaktor zu verteilen (Fig. 11). Daraus resultiert eine räumlich homogene Gassorption und -desorption, die insbesondere in der Zellkulturtechnik erwünscht ist.

Vorzugweise soll die Konstruktion eine variable und einfache Zusammensetzung der Einzelteile ermöglichen. So sind vorzugsweise die Rotorarme einzeln zu entnehmen. Daraus ergibt sich die Möglichkeit, die Membranschläuche vor der Montage auf den Rotor auf die Armpaare zu wickeln. Die Armpaare lassen sich somit auch einzeln vom Rotor demontieren. Um die separate Wicklung der Membranschläuche auf die einzelnen Armpaar zu ermöglichen, können Wickelhilfen konstruiert werden.

Im Reaktor angebrachte Tauchrohre (z.B. zur Zufuhr von Medium oder Lauge / Säure oder zum Abziehen von Ernte oder zur Probenahme) verringern das Reaktorvolumen, welches vom Rotor genutzt werden kann. Damit geht möglicherweise z.B. eine Reduktion der Membranfläche einher, die im Reaktor untergebracht werden kann. Eine Möglichkeit, dem zu begegnen ist die Integration von Tauchrohren oder auch Sonden oder sonstigen Reaktoreinbauten in den Rotor. Dadurch erfolgt ein Mitbewegen dieser Teile, was aber im Allgemeinen nicht nachteilig ist. Im Gegenteil kann hierdurch eine bessere Verteilung der zugeführten Substanzen oder ein repräsentativeres Abziehen von Flüssigkeit, z.B. Ernte bzw. eine repräsentativere Messung erfolgen.

Das Gewicht des Rotors ist vorzugsweise gering, damit das Massenträgheitsmoment des Rotors möglichst klein bleibt. Durch ein geringes Massenträgheitsmoment kann die bereitzustellende Leistung des Antriebes reduziert werden. Die erfindungsgemäße Vorrichtung wird deswegen vorzugsweise daher so leicht wie möglich bei ausreichender Stabilität gestaltet.

Das Verfahren und/oder die Vorrichtung zur blasenfreien Begasung von Flüssigkeiten, insbesondere in der Biotechnologie und speziell von Zellkulturen, findet vorzugsweise bei der jeweils optimalen Temperatur statt. Üblicherweise ist dies bei Mikroorganismen oder Zellkulturen die optimale Kultivierungstemperatur.

**Abbildungen**

Fig. 1: Schematische Darstellung einer rotatorisch oszillierenden Bewegung zur Be- und Entgasung von Flüssigkeiten mittels einer Membranfläche in einem Behältnis. Auf einen Rotor gewickelte Membranschläuche (1) bilden hier die Membranfläche. Diese rotiert mit der Rotorwelle (2) in beiden Rotationsrichtungen (3).

Fig. 2: Position, Winkelgeschwindigkeit und Drehmoment einer rotatorisch oszillierenden Bewegung zur Be- und Entgasung von Flüssigkeiten mittels einer Membranfläche. Auf einen Rotor gewickelte Membranschläuche bilden hier die Membranfläche.

Fig. 3:  Schematische Darstellung der Vorrichtung, gekennzeichnet durch eine Möglich- keit, die Spannung der Membranfläche σ, z.B. der Membranschläuche, zu variieren. Auf einen Rotor gewickelte Membranschläuche bilden hier die Membranfläche.

Fig. 4:  Schematische Darstellung der Vorrichtung, gekennzeichnet durch eine Möglich- keit, den Anstellwinkel der Membranfläche zu variieren. Auf einen Rotor gewickelte Membranschläuche (1) bilden hier die Membranfläche.

Fig. 5:  Schematische Darstellung der Vorrichtung, gekennzeichnet durch eine Möglich- keit, die durch den Strömungswiderstand bedingte Auslenkung der Membranfläche durch Richtelemente (4) in einer Rotationsrichtung zu begrenzen. Auf einen Rotor gewickelte Membranschläuche (1) bilden hier die Membran- fläche.

Fig. 6:  Schematische Darstellung der Vorrichtung, gekennzeichnet durch eine Möglich- keit, die Membranfläche zur besseren Durchmi- schung durch Richtelemente (4) entspre- chend zu formen und/oder auch Rührerblätter / Paddel (5) oder sonstige Vorrichtungen zur Strömungsführung und Durchmischung anzubringen. Auf einen Rotor gewickelte Membranschläuche (1) bilden hier die Membranfläche.

Fig. 7:  Schematische Darstellung der Vorrichtung, gekennzeichnet durch eine Möglich- keit, die Durchmischung dadurch zu verbessern, dass die Rotorarme um die Rotorwelle (2) in einer der Rotationsrichtungen (3) gebogen ausgeführt werden. Auf einen Rotor gewickelte Membranschläuche (1) bilden hier die Membran- fläche.

Fig. 8:  Schematische Darstellung der Vorrichtung, gekennzeichnet durch eine Möglich- keit, die Durchmischung dadurch zu verbessern, dass die Rotorarme tangential um die Rotorwelle (2) in einer der Rotationsrichtungen (3) an einer Vorrichtung (6) angebracht werden. Auf einen Rotor gewickelte Membranschläuche (1) bilden hier die Membranfläche.

Fig. 9:  Schematische Darstellung der Vorrichtung, gekennzeichnet durch eine Möglich- keit, die Durchmischung dadurch zu verbessern, dass die Rotorwelle (2) mit den beiden Rotationsrichtungen (3) exzentrisch im Behältnis angebracht wird. Auf einen Rotor gewickelte Membranschläuche (1) bilden hier die Membran- fläche.

Fig. 10:  Schematische Darstellung der Vorrichtung, gekennzeichnet durch eine Möglich- keit, die Durchmischung dadurch zu verbessern, dass die Rotorwelle (2) mit den beiden Rotationsrichtungen (3) zwar zentrisch im Behältnis angebracht wird, dann aber einen Exzenter (7) aufweist. Auf einen Rotor gewickelte Membranschläuche (1) bilden hier die Membran- fläche.

Fig. 11:  Schematische Darstellung der Vorrichtung, gekennzeichnet durch eine Möglich- keit, die Membranfläche pro Volumen möglichst gleichmäßig um die Rotorwelle (2) mit den beiden Rotationsrichtungen (3) zu verteilen. Auf einen Rotor gewickelte Membranschläuche (1) bilden hier die Membranfläche.

Fig. 12:  Schematischer Aufbau und Foto einer bestehenden Ausgestaltung der Erfindung ohne aufgewickelte Membranschläuche.

Fig. 13:  Foto eines Beispiels der Aufwicklung der Membranschläuche auf die Rotorarme.

Fig. 14:  Darstellung des Stofftransportkoeffizienten $k$ bei Verfahren und Vorrichtung mit Membranschlauch im Vergleich zu Membranschlauch auf herkömmlichem Schlauchstator in Abhängigkeit von dem volumenspezifischen Leistungseintrag $P/V$.

Fig. 15:  Darstellung des volumenspezifischen Leistungseintrages $P/V$ für verschiedene Ampli- tuden $y_{max}$ in Abhängigkeit von der Äquivalentumdrehungszahl $f$.

Fig. 16:  Darstellung des volumen- und bewegungsspezifischen Leistungseintrages $P_B/V$ für ver- schiedene Amplituden $y_{max}$ in Abhängigkeit von der Äquivalentumdrehungszahl $f$.

Fig. 17:  Darstellung der bewegungsspezifischen Newton-Zahl $Ne_B$ für verschiedene Amplitu- den $y_{max}$ in Abhängigkeit von der Reynolds- Zahl $Re$.

Fig. 18:  Darstellung des Stofftransportkoeffizienten $k$ mit Y-förmigen Rotorarmen für verschiedene Amplituden $y_{max}$ in Abhängigkeit von dem vo- lumenspezifischen Leistungseintrag $P/V$.

**Bezugzeichen:**

1- Membranschläuche
2- Rotorwelle
3- Rotationsrichtung
4- Richtelemente, mit denen die Auslenkung der Membranschläuche in einer Rotations- richtung begrenzt wird
5- Rührer
6- Vorrichtung zur tangentialen Anordnung der Rotorarme
7- Exzenter in Rotorwelle
8- Spannvorrichtung zur Einstellung der Membranschlauchspannung
9,10- Vorrichtungen oben und unten zur sternförmigen Aufnahme der Wickelarme, auf welche die Membranschläuche gewickelt werden
σ- Schlauchspannung

**Beispiel:**

Im Folgenden werden eine besondere Ausgestaltung der Erfindung und deren konstruktionstechnische Aspekte detailliert, ohne sich darauf zu begrenzen.

Fig. 12 zeigt den schematischen Aufbau und ein Foto des Rotors ohne aufgewickelte Membranschläuche. Die elementaren Konstruktionsteile sind dort abgebildet.

Um eine Tangentialanströmung der Membranschläuche zu gewährleisten, erfolgte die Anbringung der Membranschläuche quer zur Strömungsrichtung. Dies wurde dadurch realisiert, dass über dem Reaktorboden und unter dem Flüssigkeitsspiegel im Reaktor jeweils eine sternförmige Vorrichtung konzeptioniert wurde. Die Membranschläuche werden über die je 8 Rotorarme der in Fig. 12 gezeigten Vorrichtungen 9 und 10 gewickelt.

Fig. 13 zeigt hierfür ein Beispiel einer möglichen Aufwicklung von Membranschläuchen auf die Arme des Rotors. Die Membranschläuche sind dabei ohne Abstand auf die Wickelarme gewickelt. In diesem Beispiel sind auf jeden Arm ein 25 m langes Schlauchstück und ein 12,5 m langes Schlauchstück gewickelt. Dort, wo ein neues Schlauchstück auf dem Rotorarm beginnt, ergibt sich bei dieser Wicklung zwangsläufig eine Lücke.

Erfolgt jetzt eine Drehung des Rotors, so werden die Membranschläuche durch das Fluid im Reaktor bewegt und dadurch tangential angeströmt. Hinsichtlich der Anströmung der Membranschläuche ist zu beachten, dass die Anströmung bei gleicher Winkelgeschwindigkeit in Abhängigkeit von der Position des Membranschlauches mit zunehmender radialer Entfernung von der Rotorwelle im Allgemeinen besser wird. Grund hierfür ist die gleichermaßen zunehmende Umfangsgeschwindigkeit. Ziel muss es also sein, möglichst viele Membranschläuche möglichst weit außen bei guter Anströmung zu installieren. Eine Möglichkeit, diesem Anspruch gerecht zu werden, besteht darin, die Anzahl der Arme um die Welle zu erhöhen. Negativ wirkt sich eine Erhöhung der Anzahl der Arme allerdings sowohl auf die Durchmischung als auch auf die Anströmung der Membran aus (Schaffung von weniger durchmischten Kompartimenten wischen den Armen). Hinzu kommt, dass unter der zunehmenden Anzahl der Arme die Handhabung des Rotors beim Auf- und Abwickeln der Schläuche sowie beim Ein- und Ausbau leidet. Auch die Befestigung der Arme an der Welle gestaltet sich mit größerer Anzahl der Arme aus Platzgründen zunehmend problematisch. Hinsichtlich der oben geschilderten Aspekte erschien die Anzahl von acht Armen als ein sinnvoller Kompromiss.

Das System aus Fig. 12 weist vorzugsweise Freiheitsgrade hinsichtlich der Einstellung der Membranschlauchspannung auf. Die Schlauchspannung sollte variabel sein, um auf die Stofftransportleistung Einfluss nehmen zu können. Die freischwingenden und somit besser umströmten Schläuche sollten einen besseren Stofftransport gewährleisten. Durch Vergrößerung des vertikalen Abstandes zwischen den Vorrichtungen zur Aufnahme der Rotorarme lässt sich die Schlauchspannung variieren. Eine Feineinstellung der Schlauchspannung wird nach Lösen der Spannschrauben bis auf die im Spannblock über die Verdrehung der Schrauben im Spannblock ermöglicht.

Die Schlauchspannung stellt, wie bereits oben angesprochen, eine variable Größe dar. Aus der Reduzierung der Schlauchspannung ergibt sich das Problem der Fixierung der Membranschläuche auf den Armen. Eine große Krafteinwirkung auf die Schläuche könnte bei geringerer Schlauchspannung zum Abgleiten der Membranschläuche von den Armen führen. Um diesem Problem zu begegnen, wurde die Oberfläche der Arme mit einem Außengewinde versehen. Hierbei war darauf zu achten, dass die aufgewickelten Membranschläuche durch etwaige Grate des Gewindes nicht zu Schaden kommen. Ferner bietet das Außengewinde auf den Wikkelarmen die Möglichkeit, die Schlauchwicklung zu variieren. Bei der Aufwicklung der Schläuche könnte z.B. nur jede zweite oder dritte Gewindevertiefung genutzt werden. Hierdurch ist die Einstellung eines definierten Abstandes zwischen den einzelnen Membranschläuchen möglich.

Schon bei dem oben erwähnten Patent EP 0172478 A1 zeigt sich, dass die Durchmischung bei einem derartigen System nicht unproblematisch ist. Hinsichtlich der Durchmischung ist die Bewegungsführung bei dieser Erfindung aufgrund der oszillierenden Rotorbewegung günstiger. Eine Möglichkeit, die axiale Durchmischung zu forcieren, ist das Anstellen der Membranfläche. Durch beliebiges radiales Verdrehen von mindestens einer Vorrichtung 9 und 10 aus Fig. 12 lässt sich der Anstellwinkel der Membranschläuche verändern (vgl. auch Fig. 4). Seitens der Konstruktion muss die Möglichkeit gegeben sein, die beiden Vorrichtungen unabhängig voneinander und variabel gegeneinander zu verdrehen.

Eine weitere Möglichkeit, die Durchmischung zu fördern, besteht darin, die Membranschläuche (1) einseitig gegen Auslenkung abzustützen (vgl. auch Fig. 5) oder auszuformen (z.B. einseitig bauchförmig, vgl. auch Fig. 6). Durch die Ausformung wird eine Asymmetrie der Strömungsbilder beider Bewegungsrichtungen geschaffen. Konstruktionstechnisch erlaubt der Einsatz der Vorrichtungen (4) aus Fig. 12 die Abstützung bzw. Ausformung. Diese Vorrichtungen wurden so gestaltet, dass sie sowohl in der Höhe als auch in der Ausrichtung variabel montierbar sind. Durch Lösen bzw. Befestigen zweier Madenschrauben lassen sie sich beliebig an der Welle platzieren und fixieren.

Die Konstruktion aus den in Fig. 12 gezeigten Einzelteilen soll die Handhabbarkeit vereinfachen. So sind die Rotorarme einzeln zu entnehmen. Daraus ergibt sich die Möglichkeit, die Membranschläuche vor der Montage auf den Rotor auf die Armpaare zu wickeln. Die Armpaare lassen sich somit auch einzeln vom Rotor demontieren. Um die separate Wicklung der Membranschläuche auf die einzelnen Armpaare zu ermöglichen, wurde eine Wickelhilfe konstruiert.

Das Gewicht des Rotors sollte möglichst gering sein, damit das Massenträgheitsmoment des Rotors möglichst klein bleibt. Durch ein geringes Massenträgheitsmoment kann die bereitzustellende Leistung des Antriebes reduziert werden. Seitens der Konstruktion wurde die Erfindung daher so leicht wie möglich bei ausreichender Stabilität gestaltet.

Die besondere Ausgestaltung der Erfindung dient der Begasung eines Zellkultur-Bioreaktors mit 100 L Flüssigvolumen, wobei der Reaktorinnendurchmesser 400 mm beträgt und das Höhe zu Durchmesser-Verhältnis 2:1. Die zentrische Rotorwelle besitzt einen Durchmesser von 16 mm und der Rotor einen Außendurchmesser von 360 mm. Die Wickelarme sind im Durchmesser 14 mm ausgeführt. Um das Verrutschen der Membranschläuche mit Innendurchmesser 2 mm und Außendurchmesser 3 mm zu verhindern, ist ein entsprechendes Gewinde mit Steigung 3 mm auf die Wickelarme gedreht.

Als Rotorantrieb wurde in der dargestellten Ausgestaltung der Erfindung ein Schrittmotor mit einer Maximaldrehzahl von 2500 $min^{-1}$, einem maximalen Drehmoment von 6 Nm und einer Getriebeuntersetzung von 1 : 12 eingesetzt. Um die Spannungen aus der Verbindung Getriebe und Rotor zu absorbieren, wurde das Getriebe mit dem Rotor über Faltenbalg-Kupplungen verbundene Im Folgenden sollen ausgewählte Messergebnisse der besonderen Ausgestaltung der Erfindung kurz dargestellt und diskutiert werden. Dazu wurde das Verfahren mit der oben skizzierten Vorrichtung auf den Stofftransportkoeffizienten k (für Sauerstoff) und den volumenspezifischen Leistungseintrag bei einer volumenspez. Stoffaustauschfläche a von 30 $m^{-1}$ vermessen. Als Referenz fand gleiches für den gleichen Membranschlauch auf einem herkömmlichen Schlauchstator statt (Referenz, volumenspez. Stoffaustauschfläche a von 24

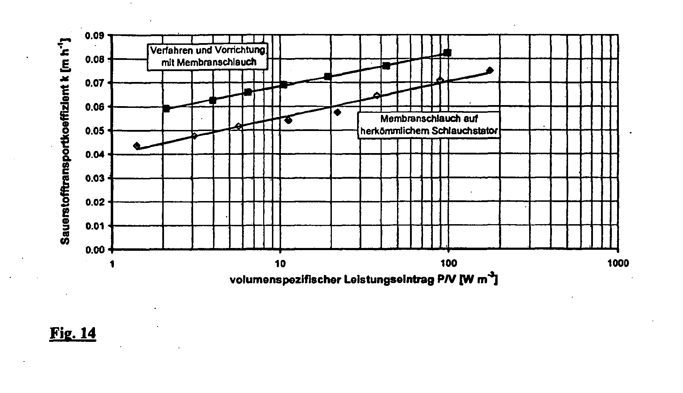

$m^{-1}$). In Fig. 14 wird der Stofftransportkoeffizient k in Abhängigkeit des volumenspez. Leistungseintrages P/V dargestellt. Der Vergleich zeigt deutlich die Steigerung des Stofftransportkoeffizienten k durch das Verfahren und die Vorrichtung. So ist der Stofftransportkoeffizient bei einem Leistungseintrag von 10 W pro $m^{-3}$ um ca. 30 % höher. Bei diesem Leistungseintrag lassen sich Steigerungen im Stofftransportkoeffizienten von ca. 70 % erzielen, wenn Membranschläuche mit Innendurchmesser 1 mm, Außendurchmesser 1,4 mm und Schlauchstücklängen von ca. 1200 mm verwendet werden (Ergebnisse nicht dargestellt).

Verbesserungen im volumenspez. Stofftransportkoeffizienten ka sind mit dem Verfahren und der Vorrichtung möglich, weil mehr volumenspez. Stoffaustauschfläche a eingesetzt werden kann. Wird der volumenspez. Stofftransportkoeffizient ka von Y-förmigen Armen (siehe Fig. 11) vergleichend zur Messreihe "Verfahren und Vorrichtung mit Membranschlauch" (siehe Fig. 14) in Abhängigkeit vom volumenspez. Leistungseintrag untersucht, so ist der volumenspez. Stofftransportkoeffizient ka bei einer zusätzlich eingesetzten volumenspez. Stoffaustauschfläche a von ca. 127% um 57% höher (Ergebnisse nicht dargestellt). Bei den Membranschläuchen mit Innendurchmesser 1 mm, Außendurchmesser 1,4 mm und Schlauchstücklängen von ca. 1200 mm lässt sich der Stofftransportkoeffizient ka bei einer zusätzlich eingesetzten volumenspez. Stoffaustauschfläche a von ca. 146 % um 224 % steigern (Ergebnisse nicht dargestellt).

Nachfolgend sind auszugsweise Untersuchungen zur Rotorbewegung präsentiert. Variierte Parameter sind hierbei Amplitude und Beschleunigung der Oszillation. Die bisher präsentierten Messreihen waren für eine Amplitude von 180° (180° hin und wieder zurück. vgl. Figur 2) aufgenommen worden. Um zu klären, ob diese anfängliche Festlegung auch die für das System geeignetste Variante darstellt, wurden Untersuchungen zu Leistungseintrag und Stofftansport einer Amplitude $y_{max}$ von 20°, 90° und 270° durchgeführt, um Vergleiche zu den bisherigen Ergebnissen zu ermöglichen. Alle Messungen sind mit Y-förmigen Rotorarmen durchgeführt worden.

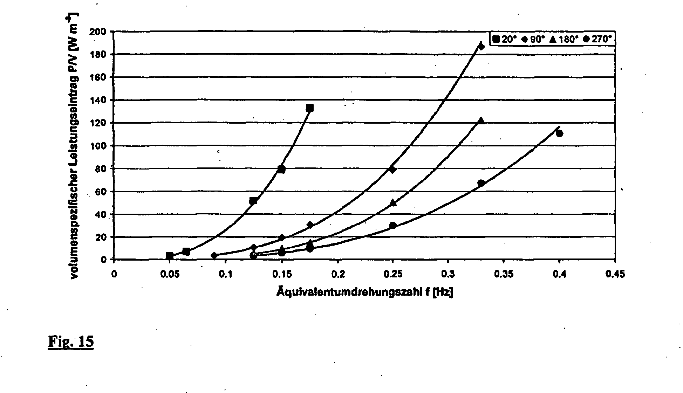

Für die von 180° abweichenden Amplituden wurden Äquivalentumdrehungszahlen in einem volumenspezifischen Leistungsbereich von 0 - 200 W pro $m^{-}$ vermessen. In Fig. 15 sind die Ergebnisse dieser Messung dargestellt.

Um die Ergebnisse besser vergleichen zu können, erfolgten nachstehende Überlegungen:

Die Äquivalentumdrehungszahl f einer Bewegung definiert sich bei einer Amplitude von 180° aus der Periodendauer T. Das ist die Zeit, die ein System benötigt, um einen Bewegungszyklus zu durchlaufen.

$$f = \frac{1}{2 \cdot T}$$

Die Äquivalentumdrehungszahl f bezieht sich immer auf die Zeit, die das oszillierende System benötigt, um 360° zu durchlaufen. Bei der bisher verwendeten Oszillation mit einer Amplitude $y_{max}$ von 180° war dies insofern einfach, weil das System bei einer Hin(+180°) - und Rückbewegung (-180°) automatisch 360° zurücklegt. Die Perdiodendauer (bzw. die Zeit für einen Bewegungszyklus) ist automatisch identisch mit der Zeit für das Durchlaufen von 360°.

Werden nun andere Amplituden als 180° verwendet, gilt dieses nicht mehr! Dann muss die Periodendauer für einen Bewegungszyklus T mit einem Faktor z, welcher die Anzahl der notwendigen Zyklen für das Durchlaufen von 360° beschreibt, auf eine Bewegung von 360° umgerechnet werden.

$$f = \frac{1}{z \cdot T} \quad \text{mit} \quad z = \frac{360°}{2 \cdot |y_{max}|}$$

Gleiches soll für den jeweilig amplitudenbezogenen Leistungseintrag gelten, welcher dem zu Folge als bewegungsspezifischer Leistungseintrag $P_B$ definiert wird.

$$P_B = \frac{P}{z}$$

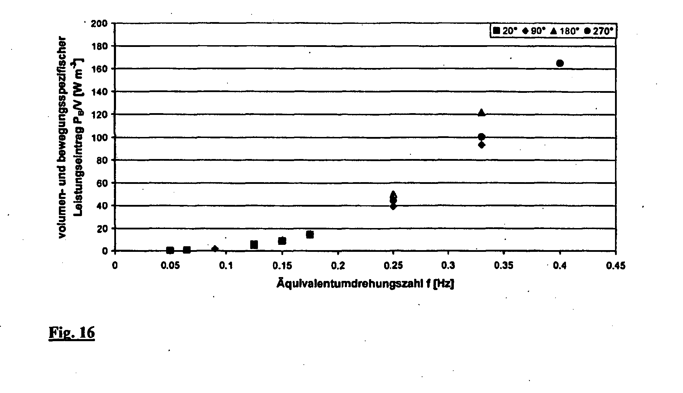

In Fig. 16 sind die Ergebnisse der Bestimmung des volumen- und bewegungsspezifischen Leistungseintrages $P_B/V$ für die verschiedenen Amplituden bezogen auf die Äquivalentumdrehungszahl f dargestellt. Es ist zu erkennen, dass alle Bewegungen eine ähnliche Leistungseintragscharakteristik aufweisen. Der volumen- und bewegungsspezifische Leistungseintrag steigt mit der entsprechenden Äquivalentumdrehungszahl. Daraus lässt sich schlussfolgern, dass die formulierten Definitionen Berechtigung haben. Werden die erhaltenen Messergebnisse auf eine Bewegung um 360° bezogen, zeigt sich, dass die durch das System eingetragene Leistung vom Wert der Amplitude unabhängig ist.

Um einen Scale-up bzw. Scale-down eines Systems durchführen zu können, ist es notwendig, das System dimensionslos beschreiben zu können. Jedes Rührsystem ist durch die dimensionslose Newton-Zahl oder auch Leistungszahl charakterisiert. Die Newton-Zahl ist abhängig vom Rührertyp und von der auftretenden Strömung. Wird die Newton-Zahl als Funktion der Reynolds-Zahl betrachtet, so ergibt sich die Leistungscharakteristik des Rührsystems. Im turbulenten Strömungsbereich stellt sich eine konstante, charakteristische Newton-Zahl ein. An dieser Stelle soll überprüft werden, ob diese Darstellung und charakteristische Beschreibung des oszillierenden Systems auf diese Weise möglich ist. Die Berechnungen erfolgten mit nachstehenden Gleichungen:

$$Re = \frac{\rho \cdot f \cdot D^2}{\eta}$$

$$Ne_B = \frac{P_B}{\rho \cdot f^3 \cdot D^5}$$

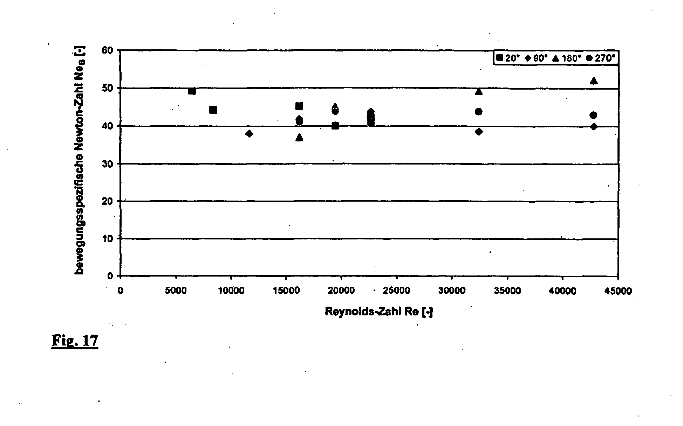

In Fig. 17 ist die Entwicklung der bewegungsspezifischen Newton-Zahl mit steigender Reynolds-Zahl dargestellt. Es zeigt sich, dass das System unabhängig von der Amplitude dimensionslos beschreibbar ist.

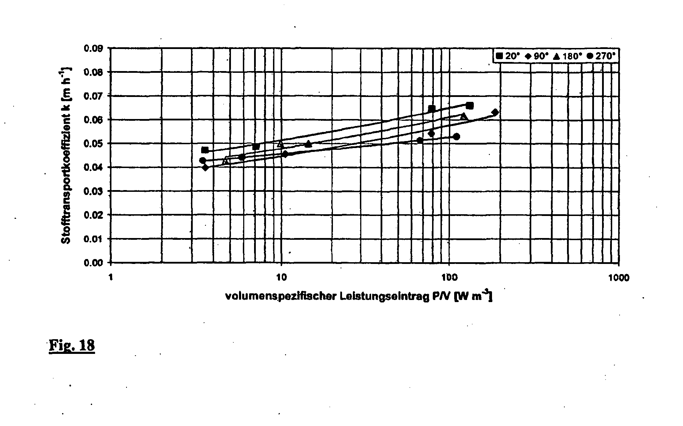

Nach der Charakterisierung des Verfahrens und der Vorrichtung in seiner Funktion als Rührsystem erfolgten die Experimente zur Charakterisierung in der Funktion als Begasungssystem. Es wurden für die drei ausgewählten Amplituden 20°, 90° und 270° Stofftransportmessungen durchgeführt und bezüglich der Ergebnisse der 180°-Oszillation vergleichend betrachtet. In Fig. 18 sind die Messergebnisse dargestellt. Es wird deutlich, dass sich alle Messergebnisse in einem Bereich von +/-10 % um die Werte für eine 180°-Oszillation anordnen. Dabei erreicht die Messreihe mit einer Amplitude von 20° das beste Resultat. Die Messreihe für eine Amplitude von 270° zeigt einen etwas anderen Verlauf als die anderen Reihen. Hier liegt eine geringere Abhängigkeit des Stofftransports vom Leistungseintrag vor.

### Patentansprüche

1. Verfahren zur Be- und Entgasung von Flüssigkeiten, insbesondere in der Biotechnologie und insbesondere von Zellkulturen, mit einem Gasaustausch über eine oder mehrere eingetauchte Membranflächen wie Schläuche, Zylinder oder Modulen, **dadurch gekennzeichnet, dass** die eine oder mehreren Membranflächen eine rotatorisch oszillierende Bewegung in der Flüssigkeit mittels einer Rotorwelle ausführen, bei der die Rotationsrichtung periodisch umgekeht wird.

2. Verfahren-nach Anspruch 1, **dadurch gekennzeichnet, dass** die Begasungsrate, der Leistungseintrag, die Mischzeit und/oder die Scherbeanspruchung durch Änderung der Bewegung, wie der Amplitude, der Äquivalentumdrehungszahl, der Beschleunigung der Membranfläche, der Verzögerung der Membranfläche, der Unterbrechung der Bewegung oder Unstetigkeiten gesteuert wird, wobei der Bewegungsablauf nicht auf wiederkehrende Muster beschränkt ist.

3. Verfahren nach einem der Ansprüche 1 oder 2, **dadurch gekennzeichnet, dass** die Begasungsrate durch Änderung der Gaskonzentration und/oder des Drucks des in den Raum innerhalb der Membranfläche einströmenden Gases oder Gasgemisches oder einer Gaskomponente gesteuert wird.

4. Verfahren nach einem der Ansprüche 1 oder 2, **dadurch gekennzeichnet, dass** die Begasungsrate durch Änderung der Gaskonzentration und/oder Druck des aus dem Raum innerhalb der Membranfläche ausströmenden Gases oder Gasgemisches oder einer Gaskomponente gesteuert wird.

5. Verfahren nach einem der Ansprüche 1 bis 4, **dadurch gekennzeichnet, dass** die Versorgung der Membranfläche mit Hilfe von flexiblen Schläuchen oder Drehdichtungen für die Zu- und Abfuhr von Gas vorgenommen wird.

6. Verfahren nach einem der Ansprüche 1 bis 5, **dadurch gekennzeichnet, dass** die Rotorarme in einer der Rotationsrichtungen gebogen um die Rotorwelle rotieren.

7. Vorrichtung zur Be- und Entgasung von Flüssigkeiten gemäß einem Verfahren nach einem der Ansprüche 1 bis 6, mit einem Gasaustausch über eine oder mehrere, in das zu be- oder entgasende Medium eingetauchte Membranflächen, die flexibel in dem zu be- und/oder entgasenden Medium angebracht sind, **dadurch gekennzeichnet, dass** die eine oder mehreren Membranflächen mittels einer Rotorwelle rotatorisch oszillierend bewegbar sind, bei der die Rotationsrichtung periodisch umkehrbar ist.

8. Vorrichtung gemäß Anspruch 7, **gekennzeichnet durch** eine Möglichkeit, die Spannung der Membranfläche, z.B. der Membranschläuche (1) zu variieren.

9. Vorrichtung gemäß einem der Ansprüche 7 oder 8 **gekennzeichnet durch** eine Möglichkeit, den Anstellwinkel der Membranfläche (1) zu variieren.

10. Vorrichtung gemäß einem der Ansprüche 7 bis 9, **gekennzeichnet durch** eine Möglichkeit, die **durch** den Strömungswiderstand bedingte Auslenkung der Membranfläche (1) in einer Rotationsrichtung zu begrenzen.

11. Vorrichtung gemäß einem der Ansprüche 7 bis 10, **dadurch gekennzeichnet, dass** die durch den Strömungswiderstand bedingte Auslenkung der Membranfläche (1) nur für einen Teil der Membran begrenzt wird.

12. Vorrichtung gemäß einem der Ansprüche 7 bis 11, **gekennzeichnet durch** eine Möglichkeit, dass die oszillierend rotierende Membranvorrichtung ohne Rotationssymmetrie ausgeführt wird.

13. Vorrichtung gemäß einem der Ansprüche 7 bis 12, **gekennzeichnet durch** eine Möglichkeit, die Membranfläche (1) zur besseren Durchmischung entsprechend zu formen und/oder Rührerblätter/Paddel oder sonstige Vorrichtungen zur Strömungsführung und Durchmischung anzubringen.

14. Vorrichtung gemäß einem der Ansprüche 7 bis 13, **dadurch gekennzeichnet, dass** die Rotorarme tangential um die Rotorwelle (2) in einer der Rotationsrichtungen (3) angebracht werden.

15. Vorrichtung gemäß einem der Ansprüche 7 bis 14, **dadurch gekennzeichnet, dass** die Rotorwelle (2) mit den beiden Rotationsrichtungen (3) exzentrisch im Behältnis angebracht werden.

## Claims

1. Method for the gassing and degassing of liquids, particularly in biotechnology, and especially of cell cultures, having a gas exchange over one or more submerged membrane surfaces such as tubes, cylinders or modules, **characterized in that** the one or more membrane surfaces execute(s) a rotating and oscillating movement in the liquid by means of a rotor shaft in the case of which the rotation direction is periodically reversed.

2. Method according to Claim 1, **characterized in that** the gassing rate, the power input, the mixing time and/or the shear load is controlled by changing the movement such as the amplitude, the equivalent rotational speed, the acceleration of the membrane surface, the deceleration of the membrane surface, the interruption of the movement or discontinuities, the movement cycle not being limited to recurrent patterns.

3. Method according to either of Claims 1 and 2, **characterized in that** the gassing rate is controlled by changing the gas concentration and/or the pressure of the gas or gas mixture or a gas component flowing into the space inside the membrane surface.

**4.** Method according to either of Claims 1 and 2, **characterized in that** the gassing rate is controlled by changing the gas concentration and/or pressure of the gas or gas mixture or of a gas component flowing out of the space inside the membrane surface.

**5.** Method according to one of Claims 1 to 4, **characterized in that** the supply of the membrane surface is undertaken with the aid of flexible tubes or rotary seals for the supply and removal of gas.

**6.** Method according to one of Claims 1 to 5, **characterized in that** the rotor arms rotate about the rotor shaft in a fashion bent in one of the rotation directions.

**7.** Apparatus for gassing and degassing liquids in accordance with a method according to one of Claims 1 to 6, having a gas exchange via one or more membrane surfaces submerged in the medium to be gassed or degassed, that are fitted flexibly in the medium to be gassed and/or degassed, **characterized in that** the one or more membrane surfaces can be moved in a rotating and oscillating fashion by means of a rotor shaft in the case of which the rotation direction can be periodically reversed.

**8.** Apparatus according to Claim 7, **characterized by** a possibility of varying the tension of the membrane surface, for example the membrane tubes (1).

**9.** Apparatus according to either of Claims 7 and 8, **characterized by** a possibility of varying the incidence angle of the membrane surface (1).

**10.** Apparatus according to one of Claims 7 to 9, **characterized by** a possibility of limiting in one rotational direction the deflection of the membrane surface (1) caused by the flow resistance.

**11.** Apparatus according to one of Claims 7 to 10, **characterized in that** the deflection, caused by the flow resistance, of the membrane surface (1) is limited only for a portion of the membrane.

**12.** Apparatus according to one of Claims 7 to 11, **characterized by** a possibility that the oscillating and rotating membrane apparatus is designed without rotational symmetry.

**13.** Apparatus according to one of Claims 7 to 12, **characterized by** a possibility of shaping the membrane surface (1) appropriately for better mixing, and/or of fitting agitator blades/paddles or other apparatuses for flow guidance and mixing.

**14.** Apparatus according to one of Claims 7 to 13, **characterized in that** the rotor arms are fitted tangentially around the rotor shaft (2) in one of the rotation directions (3).

**15.** Apparatus according to one of Claims 7 to 14, **characterized in that** the rotor shaft (2) with the two rotation directions (3) is fitted eccentrically in the container.

## Revendications

**1.** Procédé de gazage et de dégazage de liquides, notamment dans la biotechnologie et notamment de cultures cellulaires, avec un échange gazeux par l'intermédiaire d'une ou de plusieurs surfaces de membrane immergées, telles que des flexibles, des cylindres ou des modules, **caractérisé en ce que** la ou les plusieurs surfaces de membrane effectuent un déplacement oscillant en rotation dans le liquide au moyen d'un arbre de rotor, au cours duquel le sens de rotation est périodiquement inversé.

**2.** Procédé selon la revendication 1, **caractérisé en ce que** le taux de gazage, l'introduction de puissance, le temps de mélange et/ou la sollicitation au cisaillement sont commandés par modification du déplacement, comme de l'amplitude, du nombre de tours équivalent, de l'accélération de la surface de la membrane, de la temporisation de la surface de la membrane, de l'interruption du déplacement ou d'inconstances, la courbe de déplacement n'étant pas restreinte à des modèles récurrents.

**3.** Procédé selon l'une quelconque des revendications 1 ou 2, **caractérisé en ce que** le taux de gazage est commandé par modification de la concentration gazeuse et/ou de la pression du gaz ou du mélange gazeux ou d'un composant du gaz affluant dans l'espace au sein de la surface de la membrane.

**4.** Procédé selon l'une quelconque des revendications 1 ou 2, **caractérisé en ce que** le taux de gazage est commandé par modification de la concentration gazeuse et/ou de la pression du gaz ou du mélange gazeux ou d'un composant du gaz s'échappant de l'espace au sein de la surface de la membrane.

**5.** Procédé selon l'une quelconque des revendications 1 à 4, **caractérisé en ce que** l'alimentation de la surface de la membrane est assurée à l'aide de tubes flexibles ou de joints pivotants pour l'alimentation ou l'évacuation de gaz.

**6.** Procédé selon l'une quelconque des revendications 1 à 5, **caractérisé en ce que** les bras du rotor sont en rotation autour de l'arbre du rotor, de façon recourbée dans l'un des sens de rotation.

**7.** Dispositif de gazage et de dégazage de liquides selon un procédé selon l'une quelconque des revendications 1 à 6, avec un échange gazeux via une ou plusieurs surfaces de membrane immergées dans le milieu à gazer ou à dégazer, qui sont montées de façon flexible dans le milieu à gazer et/ou à dégazer, **caractérisé en ce que** la ou les plusieurs surfaces de membrane sont déplaçables en oscillation en rotation au moyen d'un arbre de rotor, pour lequel le sens de rotation est périodiquement réversible.

**8.** Dispositif selon la revendication 7, **caractérisé par** une possibilité de faire varier la tension de la surface de la membrane, par exemple des flexibles de membrane (1).

**9.** Dispositif selon l'une quelconque des revendications 7 ou 8, **caractérisé par** une possibilité de faire varier l'angle de mise en prise de la surface de la membrane (1).

**10.** Dispositif selon l'une quelconque des revendications 7 à 9, **caractérisé par** une possibilité de limiter la déviation de la surface de la membrane (1) due à la résistance à l'écoulement dans un sens de rotation.

**11.** Dispositif selon l'une quelconque des revendications 7 à 10, **caractérisé en ce que** la déviation de la surface de la membrane (1) due à la résistance à l'écoulement n'est limitée que pour une partie de la membrane.

**12.** Dispositif selon l'une quelconque des revendications 7 à 11, **caractérisé par** une possibilité que le dispositif de membrane en rotation oscillant puisse être réaliste sans symétrie rotative.

**13.** Dispositif selon l'une quelconque des revendications 7 à 12, **caractérisé par** une possibilité de conformer la surface de la membrane (1) en conséquence, pour un meilleur mélange, et/ou de monter des pales d'agitateur/des palettes ou d'autres dispositifs pour le guidage de l'écoulement et le mélange.

**14.** Dispositif selon l'une quelconque des revendications 7 à 13, **caractérisé en ce que** les bras du rotor sont montés de façon tangentielle autour de l'arbre du rotor (2) dans l'un des sens de rotation (3).

**15.** Dispositif selon l'une quelconque des revendications 7 à 14, **caractérisé en ce que** l'arbre du rotor (2) avec les deux sens de rotation (3) est monté de façon excentrique dans le récipient.

Fig. 1

**Fig. 2**

**Fig. 3**

Fig. 4

Fig. 5

**Fig. 6**

Fig. 7

**Fig. 8**

**Fig. 9**

**Fig. 10**

Fig. 11

**Fig. 12**

**Fig. 13**

**Fig. 14**

Fig. 15

**Fig. 16**

Fig. 17

**Fig. 18**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0172478 A1
- DE 8423210 U1
- DE 3535183 A1

- DE 3614712 A1
- EP 0172478 A


**In der Beschreibung aufgeführte Nicht-Patentliteratur**

- **H.-J. Henzler ; J. Kauling.** Oxygenation of cell cultures. *Bioprocess Engineering,* 1993, vol. 9, 61-75
- **R. Pörtner ; H. Märkl.** Dialysis cultures. *Applied Microbiology and Biotechnology,* 1998, vol. 50, 403-414

- **H. N. Qi ; C. T. Goudar ; J. D. Michaels ; H.-J. Henzler ; G. N. Jovanovic ; K. B. Konstantinov.** Experimental and Theoretical Analysis of Tubular Membrane Aeration for Mammalian Cell Bioreactors. *Biotechnology Progress,* 2003, vol. 19, 1183-1189